# EUROPEAN PATENT SPECIFICATION

(11) **EP 1 631 243 B1**
(45) Date of publication and mention of the grant of the patent: **15.05.2013**
(21) Application number: 04726571.5
(22) Date of filing: 08.04.2004
(51) Int. Cl.: A61K 9/00, A61K 8/98, A61Q 19/10, A61K 35/74, A61K 35/20

(54) **SEXUAL HYGIENIC COMPOSITION**
ZUSAMMENSETZUNG FÜR DIE SEXUELLE HYGIENE
COMPOSITION D'HYGIENE SEXUELLE

(30) Priority: 09.04.2003 HU 0300932; 10.03.2004 HU 0400576
(43) Date of publication of application: 08.03.2006
(73) Proprietor: Dolhay Klinika Egészségügyi KFT., 1037 Budapest (HU)
(72) Inventor: DOLHAY, Balazs, 1037 Budapest (HU)
(74) Representative: Derzsi, Katalin
(86) International application number: PCT/HU2004/000033
(87) International publication number: WO 2004/089278

(56) References cited:
- EP-A- 0 995 442
- EP-A- 1 151 754
- WO-A-94/16675
- GB-A- 2 261 372
- RU-A- 2000 111 462
- RU-C1- 2 102 062
- RU-C1- 2 137 467
- RU-C1- 2 138 955
- US-A- 4 978 528
- DATABASE WPI Week 198419, Derwent Publications Ltd., London, GB; AN 1984-116620 & JP 59 053425 A (MEIJI MILK PROD CO LTD) 28 March 1984
- DATABASE WPI Week 199940, Derwent Publications Ltd., London, GB; AN 1999-471531 & HU 215 970 B (DOLHAY B) 28 April 1999

## Description

The present invention relates to a sexual hygienic preparation, more particularly a hygienic and/or therapeutic and/or preventive and/or rehabilitative preparation.

Known and widely used types of hygienic, therapeutic, preventive, rehabilitative preparation are feminine cleansing lotions, feminine cleansing gels, feminine cleansing shampoos, feminine cleansing soaps, vaginal suppositories, vaginal pills and vaginal cleansing fluids. These can be made of concentrated liquid or water-soluble solid vaginal cleansing agents, such as effervescent tablets, granulates, or powders. The use of Adsorbent materials such as wet or dry wipes, sanitary pads, vaginal tampons in this area are also known, in addition to other vaginal suppositories, vaginal pills, lyophilized Lactobacillus preparations, lubricants and similar products with spermicidal effects. Many of these products may be considered as both sexual hygienic and therapeutic-preventive preparations. Pharmaceutical preparations used for prevention and curing of sexual diseases, as well as for the follow-up treatment thereof, also fall into this group. Typically, all the mentioned preparations act in direct contact with the genital organs. Since it is practically impossible to distinguish between hygienic, therapeutic-preventive and rehabilitative effects, for the purposes of the present description the above mentioned products are collectively referred to as sexual hygienic preparations.

Natural protection of the vagina is known to be provided by Lactobacilli. Lactobacilli produce lactic acid from glycogen in the vaginal wall and create an acidic medium with a pH of about 3.8. Shifts towards the neutral state inevitably lead to the colonization of facultative pathogenic agents, which latter are almost always present in the vagina.

In addition to the natural deterioration caused by ageing of the genital organs and by various gynaecological diseases, a number of modem products, for instance antibiotics, chemotherapeutics, hormonal contraceptives, intrauterine devices, disinfectants, traditional soaps and shampoos, and the water of swimming pools with basic pH values have side effects that decrease the efficiency of the vaginal protective mechanism. Due to the combined effect of the above mentioned factors, about a half of all patients consult gynaecologists because of vaginitis, or get hospitalized because of the consequences thereof.

A solution for restoring the natural protective mechanism of the vagina is disclosed in Hungarian patent No. 215970. There is disclosed a solid, water-soluble preparation that comprises a selective carbon source for Lactobacilli, most preferably lactose, and a buffer system for producing a pH of 3.5-4. The buffer system may consist of sodium hydrogen carbonate and citric acid. The preparation has been in use in several countries for more ten years.

Known natural therapeutic methods include irrigation with coagulated cow's milk and whey. The document JP 59,053,425 describes the application of whey for the moistening of the vagina and for restoring the self-protective mechanism thereof. Documents US 5,645,830 and US 6,004,551 disclose uro-genital preparations containing skim milk powder and Lactobacilli. The therapeutic experience of the Inventor indicates that preparations made from cow's milk are not sufficiently efficient.

It is also known that odd-toed ungulates; more particularly equids such as asses or horses, have albumin milk, similarly to humans, whereas ruminants have casein milk. It is also known that the milk of equids is most similar to mother's milk as far as the composition and characteristics thereof are concerned. The application of ass milk or horse milk (in the following collectively referred to as mare milk) for nutritional and cosmetic purposes is known. Mare milk is, however, not applied in sexual hygienic products.

The document WO95/01100 discloses a process during which preservation of ass milk is performed by fast refrigeration and subsequent slow freezing. Preserved ass milk has to be thawed before using it in skin care and beauty products. Because of technological reasons but also for reasons of comfort and convenient handling, frozen ass milk cannot be conveniently applied in sexual hygiene products.

German patent DE 19,922,932 discloses a cosmetic preparation containing ass milk and vinegar. The document IT 264,637 describes the dermatological application of preparations made from the colostrum and/or milk of equids. Sexual hygienic applications are not mentioned in either of the documents.

Lysozyme, lactoferrin, lactoperoxidase, xanthine-oxidase, vitamin-binding proteins and immunoglobulines are known to be synergic parts of the complex system that protects the organism against pathogens. Lysozyme is an immunostimulant enzyme that also dissolves the cell membrane of Gram-positive microorganisms. Pure lysozyme (E1105) is a registered food preservative, produced in bulk from egg white. Egg white contains about 5,000 ppm lysozyme. Cow milk and mother's milk contain 0.13 and 400 ppm of lysozyme, respectively, with mare milk containing roughly the same amount as mother's milk. The strongest variety of lysozyme is the human-specific one, see Csapó János, Csapóné Kiss Zsuzsa: Tejtermékek a táplálkozásban [Milk products and nutrition]. Budapest: Mezögazda, 2002, p. 348.

According to the document US 2,590,121, lysozyme produced from ass milk, while much more expensive than its widely known counterpart made from egg white, has more intensive bactericidal characteristics. Owing to its higher price, lysozyme produced from ass milk has not become widespread.

The document GB 1,212,704 describes a vaginal suppository that comprises at least one surface-active fatty compound, an effervescent compound or a compound pair, with one of the pharmaceutically active ingredients being lysozyme.

Lactoferrin is the primary iron controlling agent of the living organism. It binds iron from the digestive tract and in body fluids, depriving pathogenic agents from iron and preventing micro-organisms from proliferating. Lactoferrin is present in most body fluids in mammals, including human vaginal mucus, see Cohen, M.S. et al.: Preliminary observation on lactoferrin secretion in human vaginal mucus; Am. J. Obst. & Gyn, v.157, 1122-25. Lactoferrin is produced on an industrial scale by extracting it from the milk of mammals. Best-known producers of lactoferrin include Morinaga Milk Co., Japan, BioPole, Belgium, and Avomnore-Waterford, USA.

According to US Patent No. 6,172,040, lactoferrin treatment is applied for the preservation of meat produce. Document US 6,479,627 describes the production of stable water solution of lactoferrin for e.g. ophthalmological solutions. No sexual hygienic applications are mentioned in either document.

The main objective of the present invention is to improve the characteristics of sexual hygiene products.

The invention relates to the composition comprising equid animal milk for use in the prevention and treatment of vaginits and vulvitis. The invention relates also to the non-therapeutic use of the composition comprising equid animal milk for hygiene of female genital body parts.

The extract produced from the milk of at least one equid animal (an animal belonging to the family Equidae) is used, hereinafter also referred to as mare milk. Optionally, the milk extract may be employed with therapeutically/medically acceptable additives. In the present description the term "milk" covers milk obtained in any phase of lactation, beginning with colostrum. The term ``extract" refers to chemically non-pure material(s) or mixtures thereof that can be prepared from mare milk by the application of any suitable method. Such methods are particularly suitable which preserve the essential ingredients of mare milk. Particularly preferred method is the obtaining of milk powder on relatively low temperatures, such as by freeze drying. Another preferred method uses whey of mare milk as raw material for the extract. Preparation of milk powder with other known methods is also applicable.

The invention also concerns a sexual hygienic preparation that comprises extract made from the milk of at least one equid animal, in specific cases also comprising therapeutically/medically acceptable additive(s). Further, the invention also relates to the use of mare milk or milk extract for the manufacturing of a sexual hygienic preparation, and to the use of mare milk or milk extract for the purposes of sexual hygienic treatments.

The stratified squamous keratinising epithelium that covers the outer surface of the human body undergoes gradual change along the external pudendum. Surface layers (stratum corneum, stratum lucidum, stratum granulosum) gradually disappear when approaching to the cervical orifice (Orifitium externum uteri). Thus, non-living cover layers are gradually replaced by living cell layers on the pudendum. The covering epithelia on the juxtaposed surfaces of external genitalia adapts to wet and body identical direct contacts. In this area the efficiency of mechanical, osmotic and anti-dehydration protection processes is decreased. This is often the cause for the potential formation in this area of mirror-symmetric purulent ulcers or carcinomas that are informally called "kissing ulcer" or "kissing carcinoma". This type of mirror-symmetric deformation occurs less frequently on other skin surfaces due to added protection provided by the surface epithelial layers. The covering epithelium of the glandular epithelium also undergoes changes. Merocrine and holocrine glands occur with decreasing frequency as apocrine glands appear. More endoepithelial glands appear, and the products of glandula vestibularis minor and maior (Bartholin gland) are secreted into this region. The acidity/basicity of the surface is determined by discharge (mucus) leaking from the vagina. Shifting of the pH of vaginal discharge toward neutral seriously deteriorates the self-protection of the external pudendum. Thus, products designed to cleanse and protect other bodily surfaces may have detrimental effects on the hygiene and protection of intimate areas.

Surprisingly, it has been found that adding to known sexual hygiene ingredients an extract made from the milk of at least one equid animal, the resulting preparation will have an improved efficiency in preventing, curing and in the follow-up treatment of vaginitis and vulvitis. The preparation can help maintain eubacteriosis, that is the bacterial symbiosis that best protects the lower genital tract against pathogenic effects. Using only some selected purified ingredients of the milk did not produce the same results. The exact mechanism of this beneficial effect is not known in detail, but it is believed that the following facts provide some explanation, without restricting the invention to these theories.

The smegma under men's foreskin contains lysozyme that provides natural protection which is lacking in case of circumcised men, who (and also their sexual partners) are less protected against sexually transferred diseases such as those caused by HIV viruses (Parkash et al: Sub-Preputial Wetness - Its Nature; Ann. Nat. Med. Sci. India, 1982, V.18, 3, 109-112; Lee-Huang S. at al: Lysozyme and RNases as anti-HIV components in beta-core preparations of humanchorionic gonadotropin; Proc. Nat. Acad. Sci. USA, 1999, 96/6, 2678-2681). It was recognised that this natural protection can be substituted by a lysozyme-containing preparation that is applied to the penis or administered into the internal female genital organ.

Because the effect of lysozyme is maximal in a medium having a pH value about 6, this protective effect is not so intensive in the acidic medium (pH about 3.8) of a healthy vagina. This means that lysozyme is more "passive" if administered into the healthy female genital organ. However, in case of any local breakdown of the natural protection provided by lactic acid - which may cause an increase of the pH value of the vagina - the bactericidal and immunostimulant effect of lysozyme contained by the preparation according to the invention will be more dominant precisely when and where it is needed. The bactericidal and immunostimulant effect of lysozyme is described by Parkash et al. and by Lee-Huang S. at al in the litterature cited above.

It has also been found that the protective effect of the inventive sexual hygienic preparation is further enhanced if an extract containing lysozyme and/or lactoferrin, produced from the milk of equids and lysozyme and/or lactoferrin of alternative origin are simultaneously contained therein.

The concentration of free amino acids in mare milk is a multiple of the concentration that can be measured in cow's milk. As much as 11-21 % of whey proteins of mare milk are immunoglobulines, of which only traces occur in cow's milk (Csapó János, Csapóné Kiss Zsuzsa: Tejtermékek a táplálkozásban [Milk products in nutrition]. Budapest: Mezögazda, 2002). Immunoglobulines contained in mare milk perform the neutralization of toxins produced by bacteria and of toxic proteins released from dead bacteria. Thus, if the above extract is added to sexual hygienic preparations, then the intensity of hyperaemia in the connective tissue of the vaginal wall, potentially leading to congestion, the interstitial oedema and lymphostasis and the reactions of connective tissue producing subjective symptoms can all be successfully decreased or made less intensive.

Traditional industrial milk processing is carried out at high temperatures and pressures, resulting in the loss of several active ingredients present in native milk. Therefore, for the purposes of the present invention it is suggested that the milk is processed at a temperature under 40 °C when obtaining the extract. Preparation of an extract from mare milk on low temperatures is known per se. For example, the company Equi Libre of Nordsvea, Norway, produces mare milk powder with freeze drying (see at www.hoppemellc.no).

It has been found that the ingredients of the extract made from the milk of equids synergically complement the antipathogenic effect of the majority of traditionally used active ingredients. Thereby the self-protective and regenerative abilities of squamous epithelium suffering from hormone deficit or inflammation are significantly increased. Surprisingly, this synergic effect is noticeable already at very low added amounts of ass milk preparation (as low as 0.2 weight percent relative to solid material content).

In a preferred form the extract is milk powder, or powder prepared from acidified milk (sour milk) or whey. The extract may also be a concentrate in a liquid or semi-liquid form. In a preferred embodiment, the mare milk extract forms 0.01-20 weight percent of the solid material content of the preparation according to the invention. Preferred embodiments of the preparation are formulated in various forms of personal care products, otherwise known per se. The inventive composition may be applied to and used with adsorbent products, where the adsorbent material of the products contain the sexual hygienic preparation according to the invention. However, it is common for all products that they ensure or at least facilitate the direct contact of the active ingredients of the preparation with the affected area of the human body. These areas are primarily the genital organs, particularly the vagina. The preparation is also applicable for sexual hygiene products which are used by men.

The following non-limitative examples illustrate potential compositions and testing methods of the preparations according to the invention.

### Example:

A) 2000 effervescent tablets (M.f.l.a tablettae No MM) were prepared according to a known method from 750 mg sodium hydrogen carbonate, 900 mg lactose, and 10 mg powder mad of ass milk. The ass milk powder had been prepared at a temperature below 40 °C, using a known method of freeze drying.
B) 2000 effervescent tablets (M.f.l.a tablettae No MM) are prepared from the ingredients of Example 1/A, but without ass milk powder.
Tablets A and B could not be distinguished from each other either by appearance or by dissolution characteristics.

### Study 1

All patients taking part in the study, all between 20 and 48 years of age, had been treated with Colpo-Cleaner Iodine. "Colpo-Cleaner iodine effervescent tablet for making solutions for vaginal irrigation" is a registered medical product under Hungarian registration number OGYI T 6107. Patients were divided into two groups and matched according to the cause of their vaginitis, their age and according to their even or odd day of their date of birth. As follow-up treatment, members of both groups applied vaginal irrigation (douche) with freshly prepared, lukewarm fluid. Of 53 patients who returned for controlling consultation, 25 used tablet A and the other 28 patients used tablet B for making the irrigation fluid. Both groups applied Colpo-Cleaner irrigators (produced by Dolhay Kft., Hungary) For the first three days of the study patients irrigated (douched) 3 times a day, while during the following four-day period one irrigation was carried out each day. At the end of this period, patients shared their experiences with a gynaecologist, with the results being evaluated by a third person.

The simultaneous, matched double-blind study resulted in that, while members of the group of 25 experienced the ceasing of discomfort on the average after 4.4 days, the other group experienced the same after 5.9 days on the average.

### Study 2

58 women participated in the study. All participants were older than 52 years and all had a feeling of vaginal dryness of varying intensity before the study started. The tablets A and B of Example 1 were applied for irrigation once a day with the Colpo-Cleaner irrigator, dissolving before the tablets in lukewarm liquid. Women with odd birthdates used tablets A, while women with even birthdates used tablets B. After 5 weeks ±10 days 35 of the original participants returned for control consultation, out of which 18 had been applying tablet A and 17 tablet B. Test evaluation was carried out by a third person, who established that the randomized double-blind study showed detectable difference between the two groups. Of the group using tablet A one person, while of participants of the group using tablet B four persons said that the feeling of vaginal dryness had not become less intensive during the irrigation period. The initial irritation/itching caused by the irrigation liquid has not ceased in the case of three participants using tablet A, while the others mentioned a slight remnant feeling of irritation/itching only after having been asked targeted questions. Of the group using tablet B six women said that the feeling of irritation/itching had not ceased, while to targeted questions six other women responded that a dim feeling of irritation/itching remained.

Studies 1 and 2 showed that the application of tablet A, containing a very small amount of ass milk preparation, lead to better results. Due to the relatively small number of participants, significance tests have not been carried out.

Preparations according to the invention may be produced with compositions different from the ones illustrated in the examples using various known additives and in the form of powder, granulate, or tablets. Liquid or paste-like forms of the preparation can be produced applying liquid extracts. The extract can be made from the milk of ass or some other equid animal. Studies has shown that the difference in the chemical composition of the milk of various horse races and ass is barely discernible, if at all (See Csapó J. et al: A kanca tejének összetétele (The composition of mare's milk); Állattenyésztés és Takarmányozás, 1993, 42.2, 131-146, Hungary). What more, not only various horse races, but all equid animals produce milk which is very similar in composition. See for example: Oftedal OT, Jenness R: Interspecies variation in milk composition among horses, zebras and asses (Perissodactyla: Equidae), J Dairy Res. 1988 Feb;55(1) pp. 57-66. In this study, milk samples of four species of wild equids (onager, Equus hemionus onager; mountain zebra, E. zebra hartmannae; plains zebra, E. burchelli; Przewalski horse, E. caballus przewalskii) and two domesticated equids (ass, E. asinus; pony, E. caballus) were analysed. It was concluded that these closely related species produce milks that are nearly identical in gross composition and that the domestic horse is a representative model for the study of equid lactation. Accordingly, mare milk extract from all types of equid animals should be suitable for the purposes of the present invention.

The mare milk extract content in the various compositions may be different from those specified in the above example. In specific cases medically/therapeutically acceptable additives can also be used. The extract should most preferably contain 0.2-500 mg of mare milk powder per portion. The amount of added extract can be between 0.01-20 weight percent relative to total solid material content. The extract is usually prepared by processing milk below 40 °C, for instance by lyophilisation, but the treatment can be carried out at a higher temperature if necessary. The solidified extract can either be milk powder, or for instance, powder prepared from sour milk or whey, where the acidified (sour) milk can be produced by natural enzymatic and/or chemical process, and the powder produced form the sour milk can be prepared from the coarser-dispersion fraction and/or the re-neutralized whey.

The preparation containing the equid milk extract may further contain additional lysozyme and/or lactoferrin. The lysozyme and/or lactoferrin may also have other origin, i.e. it may be prepared from other substance than mare milk. Further additives (primarily as stabiliser) may be 0.01 weight percent pectin and/or 0.1-0.5 weight percent sorbic acid and/or 0.1-0.5 weight percent ethylenediaminetetraacetic acid or their derivatives. The extract may be contained in known adsorbent materials, such as wet or dry wipes, sanitary pads, vaginal tampons, the primary function of which can be advantageously complemented by the extract.

A significant advantage of the preparations according to the invention is that they restore faster the epithelial layers and the acidic coating destroyed by inflammation, so restitutio in integrum can be earlier achieved. It is also advantageous that the inventive preparations prevent the feeling of vaginal dryness felt by menopausal women with greater efficiency. It is believed that these advantageous effects are caused by the active ingredients contained in the extract.

The active ingredients of the extract synergically complement the antipathogenic effect of original agents. The contents of the mare milk extract nourish and protect natural Lactobacilli and also the injured parts of epithelium, and enhance the protective, preventive and therapeutic effect of the preparation, as well as the efficiency of the follow-up treatments.

As an additional advantage, the home use of the inventive vaginal douche preparations is straightforward, simple and efficient.

## Claims

1. A composition comprising equid animal milk for use in the prevention and treatment of vaginitis and vulvitis.

2. A composition according to claim 1 comprising milk extract of at least one equid animal, obtained in the course of lactation optionally combined with therapeutically acceptable additives.

3. A composition according to any of claims 1 or 2 comprising an extract processed at a temperature below 40 °C.

4. A composition according to any of claims 1-3 wherein the extract is milk powder or whey powder.

5. A composition according to any of claims 1-4 comprising both an extract prepared from the milk of equid animals containing lysosime and/or lactoferrin, and lysosime and/or lactoferrin of alternative origin.

6. A composition according to any of claims 1-5 comprising 1-0,5 weight percent ethylenediaminetetraacetic acid or a derivate thereof and/or 0,1-0,5 weight percent sorbic acid or a derivate thereof and/or 0,01-2 weight percent pectin as additive.

7. A composition according to any of claims 1-6 formulated as effervescent tablet or effervescent powder, comprising a material serving as a selective carbon source for Lactobacilli and a buffer system suitable for maintaining a pH of 3,5-4,3.

8. A composition according to claim 7 formulated as effervescent tablet or effervescent powder, a single dose of which comprises 2285 mg citric acid, 750 mg sodium hydrogen carbonate, 900 mg lactose and 0.2-500 mg mare milk powder, and optionally therapeutically acceptable addittive(s).

9. The non-therapeutic use of the composition comprising equid animal milk for hygiene of female genital body parts.

10. The use according to claim 9 comprising milk extract of at least one equid animal, obtained in the course of lactation.

11. The use according to claim 9 or 10 wherein the equid animal milk is contained by a feminine cleansing gel, feminine cleansing shampoo, feminine cleansing soap, vaginal suppository, vaginal pill, a vaginal cleansing liquid, concentrate, water-soluble solid vaginal cleansing preparation, effervescent tablet, granulate, powder, lyophilized Lactobacillus preparation, spermicide preparation.

12. The use according to any of claims 9-11 wherein the equid animal milk is contained by an adsorbent material, dry or wet wipe, sanitary pad, vaginal tampon.

13. The use according to any of claims 9-12 comprising an extract processed at a temperature below 40 °C.

14. The use according to any of claims 9-13 wherein the extract is milk powder or whey powder.

15. The use according to any of claims 9-14 comprising both an extract prepared from the milk of equid animals containing lysosime and/or lactoferrin, and lysosime and/or lactoferrin of alternative origin.

16. The use according to any of claims 9-15 comprising 1-0,5 weight percent ethylenediaminetetraacetic acid or a derivate thereof and/or 0,1-0,5 weight percent sorbic acid or a derivate thereof and/or 0,01-2 weight percent pectin as additive.

## Patentansprüche

1. Zusammensetzung, umfassend Milch von Einhufertieren für die Verwendung bei der Vorbeugung gegen und Behandlung von Vaginitis und Vulvitis.

2. Zusammensetzung nach Anspruch 1, umfassend einen Milchextrakt von mindestens einem Einhufertier, der im Lauf der Laktation erhalten wurde, gegebenenfalls in Kombination mit therapeutisch geeigneten Zusatzstoffen.

3. Zusammensetzung nach einem der Ansprüche 1 oder 2, umfassend einen bei einer Temperatur unter 40 °C verarbeiteten Extrakt.

4. Zusammensetzung nach einem der Ansprüche 1-3, wobei es sich bei dem Extrakt um Milchpulver oder Molkepulver handelt.

5. Zusammensetzung nach einem der Ansprüche 1-4, umfassend sowohl einen aus der Milch von Einhufertieren, die Lysosim und/oder Laktoferrin enthält, zubereiteten Extrakt als auch Lysosim und/oder Lactoferrin anderer Herkunft.

6. Zusammensetzung nach einem der Ansprüche 1-5, umfassend 1-0,5 Gewichtsprozent Ethylendiamintetraessigsäure oder ein Derivat davon und/oder 0,1-0,5 Gewichtsprozent Sorbinsäure oder ein Derivat davon und/oder 0,01-2 Gewichtsprozent Pectin als Zusatzstoff.

7. Zusammensetzung nach einem der Ansprüche 1-6, die als Brausetablette oder Brausepulver formuliert ist, umfassend ein Material, das als selektive Kohlenstoffquelle für Lactobazillen dient, und ein zum Aufrechterhalten eines pH-Werts von 3,5-4,3 geeignetes Puffersystem.

8. Zusammensetzung nach Anspruch 7, die als Brausetablette oder Brausepulver formuliert ist, von der eine Einzeldosis 2285 mg Zitronensäure, 750 mg Natriumhydrogencarbonat, 900 mg Lactose und 0,2-500 mg Stutenmilchpulver und wahlweise therapeutisch geeignete Zusatzstoffe enthält.

9. Nichttherapeutische Verwendung der Zusammensetzung, umfassend Milch von Einhufertieren, für die Hygiene von Körperteilen der weiblichen Genitalien.

10. Verwendung nach Anspruch 9, umfassend einen Milchextrakt von mindestens einem Einhufertier, die im Lauf der Laktation erhalten wurde.

11. Verwendung nach Anspruch 9 oder 10, wobei die Einhufertiermilch in einem Intimreinigungsgel für Frauen, einem Intimreinigungsshampoo für Frauen, einer Intimreinigungsseife für Frauen, einem Scheidenzäpfchen, einer Scheidenpille, einer Scheidenreinigungsflüssigkeit, einem Konzentrat, einer wasserlöslichen festen Scheidenreinigungszubereitung, einer Brausetablette, einem Granulat, einem Pulver, einer lyophilisierten Lactobazillus-Zubereitung, einer Spermizidzubereitung enthalten ist.

12. Verwendung nach einem der Ansprüche 9-11, wobei die Einhufertiermilch in einem adsorbierenden Material, einem Trocken- oder Feuchttuch, einer Binde, einem Scheidentampon enthalten ist.

13. Verwendung nach einem der Ansprüche 9-12, umfassend einen bei einer Temperatur unter 40 °C verarbeiteten Extrakt.

14. Verwendung nach einem der Ansprüche 9-13, wobei es sich bei dem Extrakt um Milchpulver oder Molkepulver handelt.

15. Verwendung nach einem der Ansprüche 9-14, umfassend sowohl einen aus der Milch von Einhufertieren, die Lysosim und/oder Laktoferrin enthält, zubereiteten Extrakt als auch Lysosim und/oder Lactoferrin anderer Herkunft.

16. Verwendung nach einem der Ansprüche 9-15, umfassend 1-0,5 Gewichtsprozent Ethylendiamintetraessigsäure oder ein Derivat davon und/oder 0,1-0,5 Gewichtsprozent Sorbinsäure oder ein Derivat davon und/oder 0,01-2 Gewichtsprozent Pectin als Zusatzstoff.

## Revendications

1. Une composition comprenant du lait d'origine animale d'équidés destinée à être utilisée pour la prévention et le traitement de la vaginite et de la vulvite.

2. Une composition selon la revendication 1, comprenant un extrait de lait d'au moins un animal équidé, obtenu au cours de la lactation, éventuellement combiné à des additifs thérapeutiquement acceptables.

3. Une composition selon l'une des revendications 1 ou 2, comprenant un extrait traité à une température inférieure à 40°C.

4. Une composition selon l'une des revendications 1 à 3, dans laquelle l'extrait est une poudre de lait ou une poudre de lactosérum

5. Une composition selon l'une des revendications 1 à 4, comprenant à la fois un extrait préparé à partir du lait d'animaux équidés contenant du lysozyme et/ou de la lactoferrine, et du lysozyme et/ou de la lactoferrine d'une autre origine.

6. Une composition selon l'une des revendications 1 à 5, comprenant 1-0,5 pour cent en poids d'acide éthylènediaminetétraacétique ou d'un de ses dérivés et/ou 0,1- 0,5 pour cent en poids d'acide sorbique ou d'un de ses dérivés et/ou 0,01-2 pour cent en poids de pectine en tant qu'additif.

7. Une composition selon l'une des revendications 1 à 6, formulée sous forme de comprimé effervescent ou de poudre effervescente, comprenant un matériau servant de source de carbone sélective pour Lactobacilles et un système tampon approprié pour maintenir un pH de 3,5-4,3.

8. Une composition selon la revendication 7, formulée sous forme de comprimé effervescent ou de poudre effervescente, dont une dose unitaire comprend 2285 mg d'acide citrique, 750 mg d'hydrogénocarbonate de sodium, 900 mg de lactose et de 0,2-500 mg de poudre de lait de jument, et éventuellement un ou plusieurs additifs thérapeutiquement acceptables.

9. L'utilisation non thérapeutique de la composition comprenant du lait d'origine animale d'équidés pour l'hygiène des parties du corps génital féminin.

10. L'utilisation selon la revendication 9, comprenant un extrait de lait d'au moins un animal équidé, obtenu au cours de la lactation.

11. L'utilisation selon la revendication 9 ou 10, dans laquelle le lait d'origine animale d'équidés est contenu dans un gel nettoyant pour toilette féminine, un shampooing nettoyant pour toilette féminine, un savon nettoyant pour toilette féminine, une ovule vaginale, une pilule vaginale, un liquide nettoyant vaginal, un concentré, une préparation nettoyante vaginale solide soluble dans l'eau, un comprimé effervescent, un granulé, une poudre, une préparation lyophilisée de Lactobacillus, une préparation spermicide.

12. L'utilisation selon l'une des revendications 9 à 11, dans laquelle le lait d'origine animale d'équidés est contenu dans un matériau adsorbant, une lingette humide ou sèche, une serviette hygiénique, un tampon vaginal.

13. L'utilisation selon l'une des revendications 9 à 12, comprenant un extrait traité à une température inférieure à 40°C.

14. L'utilisation selon l'une des revendications 9 à 13, dans laquelle l'extrait est de la poudre de lait ou de la poudre de lactosérum.

15. L'utilisation selon l'une des revendications 9 à 14, comprenant à la fois un extrait préparé à partir du lait d'origine animale d'équidés contenant du lysozyme et/ou de la lactoferrine, et du lysozyme et/ou de la lactoferrine d'une autre origine.

16. L'utilisation selon l'une des revendications 9 à 15, comprenant 1-0,5 pour cent en poids d'acide éthylènediaminetétraacétique ou d'un de ses dérivés et/ou 0,1-0,5 pour cent en poids d'acide sorbique ou d'un de ses dérivés et/ou 0,01-2 pour cent en poids de pectine en tant qu'additif.
